Europäisches Patentamt

European Patent Office

Office européen des brevets

(11) Publication number: **0 443 667 A1**

# EUROPEAN PATENT APPLICATION

(21) Application number: **91200299.5**

(22) Date of filing: **13.02.91**

(51) Int. Cl.⁵: **A61M 5/31, A61B 5/14**

(30) Priority: **20.02.90 US 481839**

(43) Date of publication of application:
**28.08.91 Bulletin 91/35**

(84) Designated Contracting States:
**BE CH DE FR GB IT LI LU NL**

(71) Applicant: **EASTMAN KODAK COMPANY**
**343 State Street**
**Rochester, New York 14650(US)**

(72) Inventor: **Columbus, Richard Lewis, c/o**
**EASTMAN KODAK COMPANY**
**Patent Department, 343 State Street**
**Rochester, New York 14650-2201(US)**
Inventor: **Porte, Johannes Jacobus**
**Patent Department, 343 State Street**
**Rochester, New York 14650-2201(US)**

(74) Representative: **Phillips, Margaret Dawn et al**
**Kodak Limited Patent Department Headstone**
**Drive**
**Harrow, Middlesex HA1 4TY(GB)**

(54) **Removable handle and means for attachment to a syringe or phlebotomy device.**

(57) Blood collecting devices, known as phlebotomy tubes, comprise evacuated tubes which are inserted into a syringe to collect a blood sample. Known tubes have gripping means, for example a gripping outer surface, built into the tube to allow the desired manipulation of the tube to be carried out. However, the presence of any extensive gripping surface may get in the way of any subsequent processing of the sample whilst still in the tube. The problem is particularly acute when when the tube needs to be reduced in size. Described herein is an arrangement in which a removable handle (40) can be connected to a phlebotomy tube (39) for insertion into a patient and its subsequent removal. The handle (40) and the tube (39) are connected to one another by means of a pivot pin (52) formed in the handle (40) which engages with an aperture (50) formed in the tube (39). A hook end (62) is formed on a bayonet member (60) carried by the handle (40), which when the handle (40) is rotated relative to the tube (39) about the pivot pin (52), latches into a recess (72) formed in a latch groove (70) on the tube (39) to prevent separation of the handle (40) and tube (39) when axial force is applied to the combined device. The off-axis joining of the handle (40) to the tube (39) renders visually apparent when sufficient unlocking by rotation of the handle (40) has occurred.

FIG. 1

This invention relates to a handle for phlebotomy devices or drug-injecting syringes, particularly those handles which are removable.

Blood collecting devices, known as phlebotomy tubes, are usually evacuated tubes which are inserted into syringes to collect blood from a vein. Typically, several such tubes are sequentially inserted into a syringe while that syringe remains injected into a patient. Thus, the tubes usually have gripping means built into the tube to allow their manipulation in the manner stated, for example, the outside of the tube may be the gripping surface. However, further processing of the tubes is needed after blood collection, and any extensive gripping portion can be objectionable if it gets in the way of such processing. Particularly this becomes true if the collecting container is reduced in size, as then a handle must be added which serves no other function.

Thus, it would be convenient if a phlebotomy tube could be provided with a removable handle which is used only when the tube is put into or taken out of the syringe.

Removable handles have been typical of drug-injecting syringes. Generally such handles involve a friction fit with the piston used to push the drug out of a prepackaged container. Examples of such a construction are disclosed in US-A-3045674, US-A-4507117, and German Patentschrift 218668. Such friction fit, however, is not a very secure engagement, and there is no surety that accidental disengaging will not occur.

To improve upon such problems of friction fits, a rotating latch mechanism for a drug injection syringe is described in US-A-4677980. This mechanism rotates 90° between an engaged connection and a disengageable connection. However, this construction renders it difficult to ascertain whether the handle is locked or not, as both conditions present the same overall appearance of the handle vis-a-vis the rest of the device. This is because the handle is rotated about a common axis with the syringe so that its apparent geometry vis-a-vis the syringe does not markedly change so as to suggest it is unlocked.

As far as can be ascertained, removable handles have never been proposed for phlebotomy devices prior to the present invention. This means that there is a tendency to discourage any reduction in size of the phlebotomy device, since the built-in gripping surface alone mitigates against size reduction.

Thus, prior to the present invention, there has been a problem in providing a removable handle for either a phlebotomy tube or a piston of a drug-injecting syringe which, by its rotation, easily tells the user whether it is engaged or disengageable.

It is therefore an object of the present invention to provide a handle construction which differs markedly in its position when it is unlocked, compared to its locked relationship with the syringe or phlebotomy device.

More specifically, in accordance with one aspect of the present invention, there is provided a blood collecting or drug-injecting device comprising:-

a reservoir in which blood is collected or in which a drug is retained prior to injection respectively, the reservoir having a longitudinal axis;

a needle connected to the reservoir and through which transmission of blood out of or a drug into a patient is effected; and

a handle connectable to the reservoir to aid insertion of the needle into the patient and its subsequent removal after blood has been collected or a drug delivered, the handle having a longitudinal axis;

characterized in that either one of the handle or the reservoir has a pivot pin formed thereon which frictionally mates with an aperture formed in the other of the handle or reservoir, the pivot pin and the aperture both being offset from and extending generally parallel to the respective longitudinal axes so as to assymetrically join the handle to the reservoir in a rotational orientation;

and in that latching means are provided for latching the handle and the reservoir together to prevent the pin and the aperture from being axially separated by opposing forces applied along the longitudinal axes.

Thus, it is an advantageous feature of the invention that a phlebotomy device or syringe can be provided with a removable handle which has a positive engagement which is geometrically indicative of its latched or unlatched condition.

It is a related advantageous feature of the invention that such a combination of handle and phlebotomy device or syringe can feature a reduction in size, except for the handle, which permits subsequent processing to proceed without the handle, using smaller apparatus.

The present invention will now be described by way of example only, with reference to the accompanying drawings in which:-

Figure 1 is a sectioned elevational view of a phlebotomy device constructed with a handle in accordance with the present invention;

Figure 2 is a sectioned view of the handle taken generally along the line 2-2 of Figure 1;

Figure 3 is a fragmentary side elevational view of the handle shown in Figure 2;

Figure 4 is an end elevational view of the phlebotomy device taken generally along the line 4-4 of Figure 1;

Figure 5 is a fragmentary side elevational view of the device shown in Figure 4;

Figure 6 is a fragmentary side elevational view showing the movement of the handle to latch it in place; and

Figure 7 is a fragmentary elevational view of the use of the handle of the invention with a piston in a drug-injecting syringe.

The invention is described particularly in reference to the preferred embodiments featuring certain phlebotomy devices and syringes for blood collection and drug injection respectively. In addition, it is useful regardless of the design of the phlebotomy device or syringe, and regardless of whether the syringe is used with drugs or some other material.

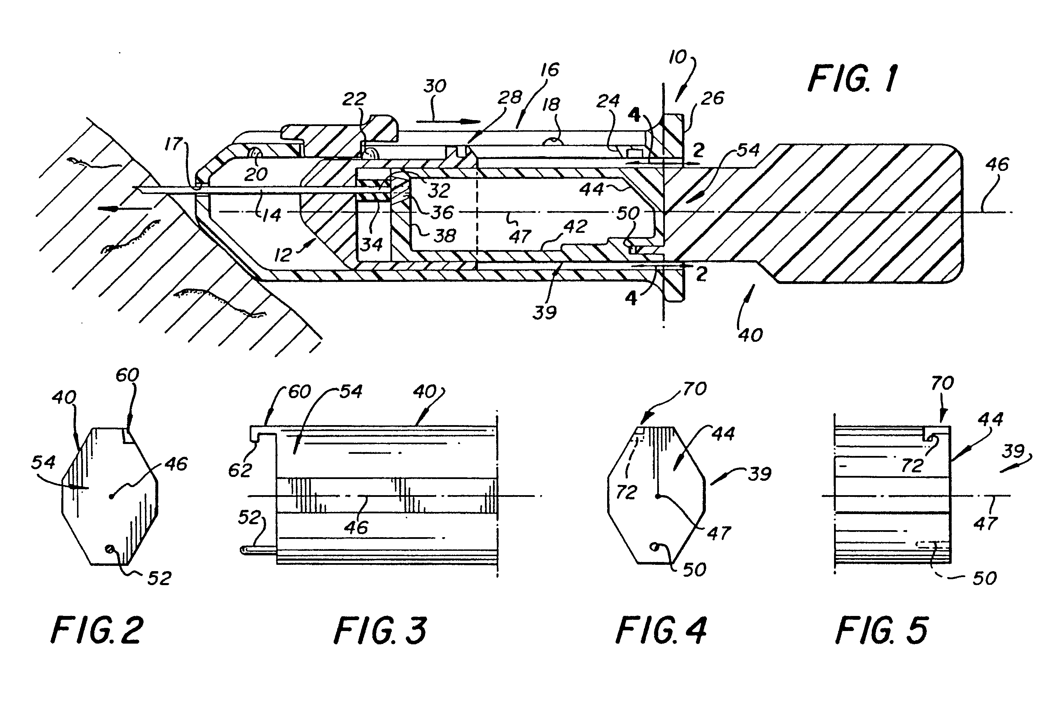

Turning first to Figure 1, any phlebotomy device 10 can be provided with a handle 40 of the invention. Preferably, device 10 comprises a needle mounting member 12 in which a needle 14 is permanently embedded, member 12 in turn being slidable mounted in a safety shield or housing 16. Such a shield 16 cooperates with member 12 as described in US Patent Application Serial No. 481838. However, such a shield 16 is optional and can be omitted. If included, shield 16 has a needle aperture 17 and recesses 18 and 20 which co-act with one or more detents 22 on member 12 to releasably hold the needle and mounting member 12 in one of two positions. In addition, locking tab 24 is formed at end 26 of shield 16, to cooperate with a latch 28 on member 12 to permanently hold the needle withdrawn after all blood is collected and the mounting member is withdrawn (in the direction indicated by arrow 30).

With or without the shield 16, needle 14 includes an end 32 which projects into member 12, with a collapsible sleeve 34 covering it. This end is designed to penetrate a septum 36 at one end 38 of a blood-collecting container 39, as is conventional. The container includes at least one liquid-confining compartment 42 and a longitudinal axis 47.

In accordance with the invention, opposite end 44 of container 39 has a handle 40 removably attached thereto. Handle 40 has an axis 46 (Figure 3) which ensures that when the handle is latched, as shown in Figure 1, axis 46 coincides with axis 47 of container 39.

To provide for a positive latching of the handle, in a manner which geometrically distinguishes the latched condition from the unlatched condition, the following features are included:

End 44 of container 39 has an aperture 50 which extends parallel to axis 47, as shown in Figure 4. A corresponding, mating pivot pin 52 projects from end 54 of handle 40 (Figures 2 and 3) and is also parallel to axis 46, in a position so as to line up with aperture 50 when axis 46 of handle 40 is aligned with axis 47. However, to ensure that the unlatched configuration of handle 40 is distinguishable from the latched one, pin 52 and aperture 50 are disposed to be offset from axes 46 and 47 respectively.

The positive latching of the handle 40 to container 39 is further achieved by a hook or bayonet member 60 mounted on handle 40, as shown in Figure 3, the handle 40 sliding into a mating groove 70 formed in end 44 of container 39 (Figure 4). Member 60 has a hook end 62 which projects into the handle toward axis 46, and cooperates with a recess 72 which also projects inwardly of container 39 towards axis 47 (Figure 5).

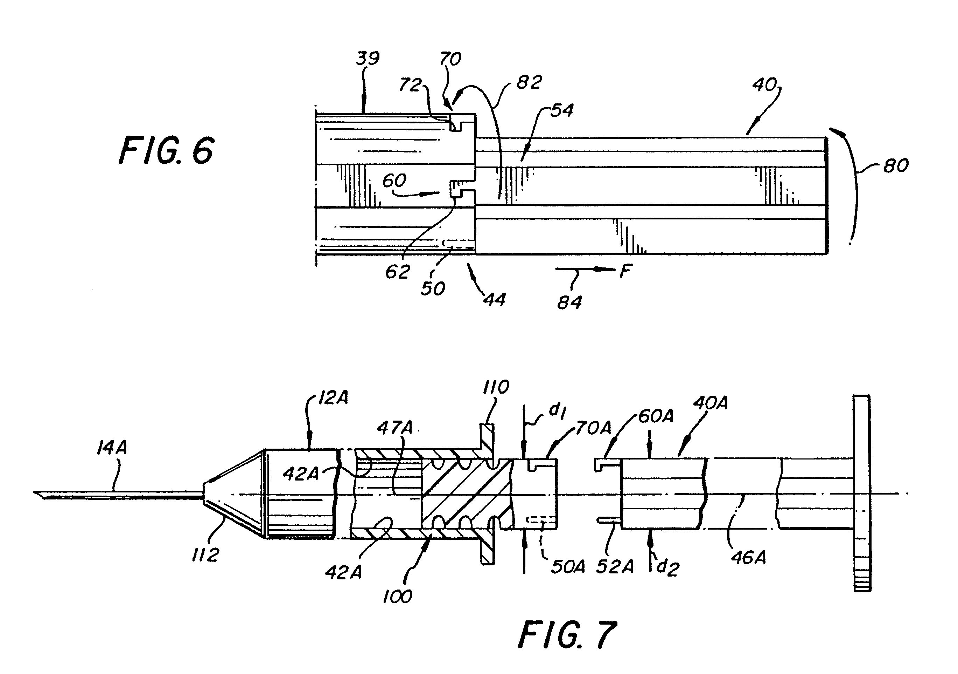

The assembly of handle 40 into its latched position is shown in Figure 6. End 54 is abutted against end 44 of container 39 so that pin 52 is inserted into aperture 50, and the handle is rotated, in the direction of arrow 80, to bring hook member 60 into groove 70 on container 39. When hook end 62 seats into recess 72, as shown by arrow 82, the latching is complete and any axially directed force F (indicated by arrow 84) is ineffective in separating handle 40 from container 39.

After blood collection is complete, handle 40 and device 39 are pulled out of housing 16, and the handle 40 is removed. Thereafter, device 39 can be further processed to achieve serum separation using conventional techniques.

Handle 40 has several advantages stemming from its construction. When it is fully latched to container 39, the latching is a positive engagement which cannot slip, as some frictional engagements are capable of doing. Furthermore, the latched and unlatched configurations are, by reason of their different axial alignments, clearly distinguishable one from the other (compare Figures 1 and 6), so that the user is able to identify which condition is present simply from the geometry. Still further, the use of the removable handle permits device 39 (Figure 1) to be greatly reduced in size to the point at which it fits totally inside housing 16, thus becoming non-manipulable without the handle 40. For example, the total length of device 39 can be reduced to only 2.5cm (1in). This is particularly useful as blood draws are reduced in volume to supply analyzers which need much less sample.

It will be readily appreciated that pin 52 and aperture 50 can be reversed (not shown) so that the pin is on container 39. Likewise, member 60 and groove 70 can be reversed, so that member 60 is on container 39.

It will also be appreciated that the removable handle will readily attach to a piston of a drug-injecting syringe, as shown in Figure 7. Parts similar to those previously described bear the same reference numeral, to which the distinguishing suffix A has been appended.

Thus, handle 40A is readily attachable and removable from a piston 100 having an outside diameter $d_1$, where the outside diameter $d_2$ of handle 40A preferably is slightly less than $d_1$. Piston 100 slides into end 110 within compartment 42A of drug-delivery syringe 12A which mounts a needle 14A at opposite end 112. The syringe construction is conventional, so that no further discussion is required.

The engagement of handle and piston is substantially the same as for the previous embodiment - pivot pin 52A is inserted into aperture 50A, and the handle rotated until member 60A latches into groove 70A in the surface of piston 100 and axis 46A of handle 40A coincides with axis 47A of piston 100. Thereafter, piston 100 can be pushed into container 39A to push the liquid contents out of needle 14A. The advantages of handle 40 enumerated above for the phlebotomy device accrue as well for handle 40A.

## Claims

1. A blood collecting or drug-injecting device comprising:-

   a reservoir (39; 39A) in which blood is collected or in which a drug is retained prior to injection respectively, the reservoir (39; 39A) having a longitudinal axis (47; 47A);

   a needle (14; 14A) connected to the reservoir (39; 39A) and through which transmission of blood out of or a drug into a patient is effected; and

   a handle (40; 40A) connectable to the reservoir (39; 39A) to aid insertion of the needle (14; 14A) into the patient and its subsequent removal after blood has been collected or a drug delivered, the handle (40; 40A) having a longitudinal axis (46; 46A);

   characterized in that either one of the handle (40; 40A) or the reservoir (39; 39A) has a pivot pin (52; 52A) formed thereon which frictionally mates with an aperture (50; 50A) formed in the other of the handle (40; 40A) or reservoir (39; 39A), the pivot pin (52; 52A) and the aperture (50; 50A) both being offset from and extending generally parallel to the respective longitudinal axes (46, 47; 46A, 47A) so as to asymmetrically join the handle (40; 40A) to the reservoir (39; 39A) in a rotational orientation;

   and in that latching means (60, 62, 70, 72; 60A, 70A) are provided for latching the handle (40; 40A) and the reservoir (39; 39A) together to prevent the pin (52; 52A) and the aperture (50; 50A) from being axially separated by opposing forces applied along the longitudinal axes (46, 47; 46A, 47A).

2. A device according to claim 1, wherein the latching means (60, 62, 70, 72; 60A, 70A) comprises a bayonet member (60, 62; 60A) formed on the handle (40; 40A) which engages with a latch groove (70, 72; 70A) formed on the reservoir (39; 39A), the bayonet member (60, 62; 60A) including a hook end (62), and the latching groove (70, 72; 70A) including a recess (72), the handle (40; 40A) being rotated relative to the reservoir (39; 39A) to cause hook end (62) to seat in recess (72) to complete the latching.

3. A device according to claim 1, wherein the latching means (60, 62, 70, 72; 60A, 70A) comprises a bayonet member (60, 62; 60A) formed on the reservoir (39; 39A) which engages with a latch groove (70, 72; 70A) formed on the handle (40; 40A), the bayonet member (60, 62; 60A) including a hook end (62), and the latching groove (70, 72; 70A) including a recess (72), the handle (40; 40A) being rotated relative to the reservoir (39; 39A) to cause hook end (62) to seat in recess (72) to complete the latching.

4. A device according to any one of the preceding claims, wherein the device is a phlebotomy tube.

5. A device according to any one of the preceding claims, wherein the device is a drug-injecting syringe.

*FIG. 1*

*FIG. 2*

*FIG. 3*

*FIG. 4*

*FIG. 5*

EP 0 443 667 A1

FIG. 6

FIG. 7

EP 0 443 667 A1

## DOCUMENTS CONSIDERED TO BE RELEVANT

EP 91200299.5

| Category | Citation of document with indication, where appropriate, of relevant passages | Relevant to claim | CLASSIFICATION OF THE APPLICATION (Int. Cl. 5) |
|---|---|---|---|
| A | DE - A - 1 491 661 (R. BLACK) * Fig. 3,4; page 8, paragraph 3 * | 1 | A 61 M 5/31 A 61 B 5/14 |
| A | US - A - 3 388 941 (J.A. MARCUS) * Totality; especially column 3, line 57 - column 4, line 13 * | 1 | |
| D,A | US - A - 4 677 980 (D.M. REILLY et al.) * Fig. 2,9,10,11,17,20; abstract * | 1 | |
| D,A | US - A - 3 045 674 (S.D. GOLDBERG) * Fig. 1-9; claims 1,2 * | 1 | |
| | | | **TECHNICAL FIELDS SEARCHED (Int. Cl.5)** A 61 B A 61 M |

The present search report has been drawn up for all claims

| Place of search VIENNA | Date of completion of the search 05-06-1991 | Examiner LUDWIG |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)